Europäisches Patentamt

European Patent Office

Office européen des brevets

(11) Publication number: **0 417 298 A1**

# EUROPEAN PATENT APPLICATION
## published in accordance with Art. 158(3) EPC

(21) Application number: 90902686.6

(22) Date of filing: 02.02.90

(86) International application number:
PCT/JP90/00127

(87) International publication number:
WO 90/08956 (09.08.90 90/19)

(51) Int. Cl.⁵: **G01N 33/53**, G01N 33/577,
C12P 21/08, C07K 1/14,
C07K 13/00, A61K 37/02

(30) Priority: 02.02.89 JP 22634/89
17.02.89 JP 36228/89
18.04.89 JP 96456/89
04.12.89 JP 314602/89

(43) Date of publication of application:
20.03.91 Bulletin 91/12

(84) Designated Contracting States:
BE CH DE DK FR GB LI SE

(71) Applicant: TEIJIN LIMITED
6-7, Minamihonmachi 1-chome Chuo-ku
Osaka-shi Osaka 541(JP)

(72) Inventor: KOIKE, Yukiya
399-1-102, Miya Hino-shi
Tokyo 191(JP)
Inventor: SUMI, Yoshihiko
4-40-49, Toyoda Hino-shi
Tokyo 191(JP)
Inventor: ICHIKAWA, Yataro
2-11-7, Kotesashi-cho Tokorozawa-shi
Saitama 359(JP)

(74) Representative: Kraus, Walter, Dr. et al
Patentanwälte Kraus, Weisert & Partner
Thomas-Wimmer-Ring 15
W-8000 München 22(DE)

(54) DETECTION OF HUMAN TISSUE FACTOR ACTIVATOR.

(57) This invention relates to an immunoassay of a human tissue factor activator originating in the human urine by utilizing an antihuman tissue factor activator monoclonal antibody, which is useful for diagnosing, for example, renal diseases. The tissue factor activator originating in the human urine is a new material. The invention also discloses the antihuman tissue factor activator monoclonal antibody to be used in the above assay and hybridoma cells which produce said antibody.

# METHOD OF DETECTING HUMAN TISSUE FACTOR ACTIVE SUBSTANCE

## Technological Field

This invention relates to a method of detecting a human tissue factor active substance. More specifically, this invention relates to a method of detecting a human tissue factor active substance in human urine by utilizing an anti-human tissue factor active substance monoclonal antibody. Furthermore, this invention relates to an anti-human tissue factor active substance monoclonal antibody which can be utilized in the above method, hybridoma cells which are produced by the monoclonal antibody, the use of the monoclonal antibody in the purification of a human tissue factor active substance, and to a substantially pure human tissue factor active substance itself.

## Background Technology

Human tissue factor (Tissue Factor, to be sometimes abbreviated as "TF" hereinafter is also called tissue thromboplastin, and is known to show an important function as an initiating substance of extraneous coagulation. It is a substance which forms a complex with Factor VII, and Factor Xa and Factor IX. Tissue Factor (TF) is a glycolipoprotein composed of a lipid moiety and a protein moiety (apoprotein), and the development of its activity requires the existence of both. Apoprotein is a glycoprotein having a molecular weight of about 50,000 and is considered to be a kind of membrane protein. In intact cells, the tissue factor is considered to exist as covered with the surface. Particularly, the issue factor is exposed to the cell surface by damages of tissue and vessels in cancer and so on, and intravascular coagulation occurs easily. It is reported that TNF (Tumor Necrosis Factor) or IL (Interleukin), or cytokines sti mulates certain cells, and on the cell surface, activation of tissue factor takes place [(P.R. Conling, C. S. Greenberg, and J. B. Weinberg: Blood, vol 72, No. 1, 128 - 133 (1988)].

Recently, a cDNA clone encoding a human tissue factor apoprotein was isolated, and the primary structure of the protein was elucidated [see (E. K. Spicer et al: Proc. Natl. Acad. Sci., USA, vol. 84, 5148-5152 (1987)].

It has been made clear that a human tissue factor apoprotein is water-insoluble, but when it is digested with an enzyme such as trypsin, it is solubilized.

It is known that human tissue factor exists in various organs of the entire body, but occurs especially in large amounts in the lungs, brain and placenta, and vasclar endothelial cells also produce tissue factors [Colucci M. et al.: I. Clin. Invest. 71 , 1893 - 1896 (1983)].

Anticipating that blood vessels are concentrated on the kidney tissues, and metabolism and extretions are conducted actively and particularly in patients of kidney diseases who undergo damages in the kidney tissue itself and the blood vessel concentrated on it, tissue factors are released in considerably large amounts, the present inventors prepared anti-human tissue factor monoclonal antibodies using human tissue factors separated from human placenta in order to detect the tissue factors in the urine and attempted to detect the tissue factors in the urine. As a result, it has been found that a substance showing the activity like tissue factor binding to the monoclonal antibody (to be referred to as tissue factor (TF) active substance. The present inventors found that the amount of this substance differs between healthy persons and patients of kidney diseases (nephritis). Accordingly, to measure the amount of this substance is useful for the diagnosis of kidney diseases (nephritis), and this finding led to the accomplishment of the present invention.

## Disclosure of the Invention

Thus, the present invention provides a method of immunologically detecting a tissue factor active substance in human urine. The immunological assay of a human tissue factor active substance in accordance with this invention can also be carried out by an immuno-precipitation method, an immunodiffusion method and an immunoelectrophoresis method using an anti-human tissue factor active substance polyclonal antibody. Generally, the following methods may utilizing an anti-human tissue factor active substance monoclonal anti-body be preferably used.

Method A:

A method of assaying a human tissue factor active substance in human urine by using two types of anti-human tissue factor active substance monoclonal antibodies which recognize different epitopes of human tissue factor active substance, two of the antibodies being used as a primary antibodies fixed to an insoluble carrier, and the other being used as a secondary antibody.

Method B:

A method of assaying a human tissue factor active substance in human urine by a competitive method by adding to human urine a labelled human tissue factor active substance and an anti-human tissue factor active substance monoclonal antibody.

Method C:

A method of detecting a human tissue factor active substance in human urine, which comprises adding a known amount of a labelled anti-human tissue factor active substance monoclonal antibody to human urine to react it with a human tissue factor active substance that may exist in the urine, thereafter adding an insoluble carrier to which a human tissue factor active substance is fixed which may be bound to the antibody, then separating the insoluble carrier, and measuring the amount of the antibody bound to the insoluble carrier.

These methods will be described further in detail below.

Method A (sandwich method):

The immunological assaying method by the sandwich method is generally a method by which by using an antibody bound to two different sites of an antigen, the presence or absence of the antigen or its amount is determined [see Wide, Radioimmunoassay method, 199 - 206 (1970)]. The sandwich method may be carried out by performing all reactions in the liquid phase (liquid phase method), or part of the reaction may be carried out in the solid phase (solid phase method). In the method of this invention, it is carried out advantageously by the liquid phase method because of the ease of operation.

The anti-human TF active substance monoclonal antibodies used in this method may be not particularly limited if they are two types of monoclonal antibodies which recognize different epitopes of human TF active substance. Specific examples are anti-human TF active substance monoclonal antibodies which bind specifically to human TF active substance and which do not inhibit the blood coagulating (clotting) activity of the human TF active substance, and which have been found by the present inventors. More specifically, they include hybridoma cells FERM P-10505 (trasferred under the Butapest Treaty relating to the international recognition of the deposition of microorganisms in the Patent procedures (simply "the Butapest Treaty") on January 22, 1990, and assigned No. BP-2739 (producing monoclonal antibody GX3); FERM P-10506 (transferred to deposition based on the Budapest Treaty, and assigned FERM BP-2740 (producing monoclonal antibody GX4), and FERM P-10507 (producing monoclonal antibody EX6), and anti-human TF active substance monoclonal antibodies having equivalent binding characteristics.

In the present specification, the human tissue factors active substance include those having the same structure as TF derived from known brain and placenta, and substances which have a different structure from known TF, namely substances which induce blood coagulation in the presence of a phospholipid, and a calcium ion.

Now, the characteristics of the anti-human tissue factor monoclonal antibodies GX3, GX4 and EX6 produced by the above hybridomas will be described.

They all specifically bind to human TF active substances, and do not inhibit the blood coagulating activity of human TF active substance. Furthermore, GX3 and GX4 recognize a fragment of human TF apoprotein represented by the following formula (I)

3

```
    VPKPEWELITKFNTSKWTLNYAAVTNTTGS-NH2
       N
      Q
      V
      Y
       T
    VQISTKSGDWKSKCFYTTDTECDLTDEIVKDVK
                                    Q
                                     T
                                      Y
                                       L
                                        A
    TELYPTFEPSNEYLPEGASGTSEVNGAPYSFVR
       N
      L
     G
     Q
      P
      TIQSFEQVGTKVNVTVEDERTLVRRNNTFLSLR
                                       D
                                        V
                                         F
                                        G
                                       K
      EGKDVDILFENTNTKATKKGSSSSKWYLTYILD
       N
      Y
     C
      F
       S
       VQAVIPSRTVNRKSTDSPVECM-COOH          ... (1)
```

but do not recognize the fragment of the human TF active substance apoprotein represented by the following formula (II)

```
    H2N-GQEKGEFREIFYIIGAVVFVVIILVIILAISLHK┐
                                          │
    └CRKAGVGQSWKENSPLNVS-COOH          ... (II).
```

Furthermore, GX4 also recognizes a higher structure of the apoprotein of formula (I).

On the other hand, EX6 dies not recognize the fragment of the apoprotein of formula (1), but recognizes the fragment of the apoproerin of formula (II).

The abbreviations of the amino acids in the above formulae are in accordance with page 31 of Iwanami Physico-chemical Chemical dictionary (4th edition), and have the following meanings throughout the present specification.

N:    asparagine
D:    aspartic acid
A:    alanineR
R:    arginine
I:    isoleucine
G:    glycine
Q:    glutamine
E:    glutaminic acid
C:    cysteine

4

S:   serine
Y:   tyrosine
W:   tryptophan
T:   threonine
V:   valine
H:   histidine
F:   phenylalanine
P:   proline
M:   methionine
K:   lysine
L:   leucine

Preferred among these antibodies is an anti-human TF monoclonal antibody which does not bind to a fragment on the carboxyl group terminus side containing a domain which binds to a phospholipid of a TF apoprotein derived from human placenta obtained by treating a TF apoprotein derived from human placenta with CNBr, which binds to a fragment on the amino group terminus side not containing a domain binding to a phospholipid of TF apoprotein derived from human placenta, and which does not inhibit blood clotting activity of human TF, that is to say a monoclonal antibody of anti-human tissue factor which does not bind to a fragment of human TF apoprotein of formula (II), binds to a fragment of human TF apoprotein represented by formula (1) and does not inhibit the blood coagulation (clotting) activity of the human TF active substance.

specifically, examples include monoclonal antibodies produced by hybridoma cell FERMK BP-2739 (producing monoclonal antibody GX3) and FERMK BP-2740 (producing monoclonal antibody GX4), and anti-human TF active substance monoclonal antibodies having equivalent binding characteristics.

These antibodies may be used in the form of a complete antibody, but also as antibody fragments which maintain essential binding properties, for example, as univalent Fab, Fab' and (Fab')$_2$.

Thus, in the present specification, the term "antibody" is meant to include not only a compete form of antibody, but also its fragments.

According to the sandwich method, two types of monoclonal antibodies which recognize different epitopes of human TF active substance are properly selected from the anti-human TF active substance monoclonal antibodies, and one of them is fixed to an insoluble carrier and used as a first antibody, and the other as a second antibody. In the present method, it is especially preferred to use a monoclonal antibody (GX3) produced by hybridoma cells FERM BP-2739 or an anti-human TF active substance monoclonal antibody having equivalent binding characteristics as a primary antibody binding to an insoluble carrier, and to use a monoclonal antibody (GX4) produced by hybridoma cells FERM BP-2740 or an anti-human TF active substance monoclonal antibody having equivalent binding characteristics as a secondary monoclonal antibody.

On the other hand, immunologically inert and substantially inert solid in the aqueous media used in the present method may be used without restriction as the insoluble carriers for the primary antibodies. Examples include polymeric materials as polystyrene, polyethylene, polypropylene, polyesters, polyacrylonitrile, fluorine resins, cellulose or its derivatives, crosslinked dextran, polysaccharides and agarose, inorganic solid substances such as silica, glass and metals; and combinations of these materials.

The shape of the insoluble carrier may be various such as a tray, a sphere, a fiber, a rod, a disc, a container, a cell or test tube.

Fixation of the primary antibody to the insoluble carrier may be performed by any known methods. For example, there may be used a physical method in which an antibody and an insoluble carrier are contacted for a fixed period of time in a buffer prepared to have a suitable pH and a salt concentration to perform adsortion. There may be also a chemical method by which an antibody having introduced thereinto a SH crosslinking agent (such as SPDP, or maleimide) is reacted with an insoluble carrier having its surface treated with SH to bind the antibody through a disulfide bond (S-S).

On the other hand, the anti-human TF active substance monoclonal antibody as a secondary antibody is usually labelled. This is however not essential. Another monoclonal antibody or polyclonal antibody which binds to such a secondary antibody may be used as a tertiary antibody, and it may be labelled. In the present invention, a final detecting means is not an essential problem, and it is essential to use two kinds of anti-human tissue factor monoclonal antibodies which recognize different epitopes as two antibodies of one pair. The detecting means which are labelled and fixed by such a combination may be any means.

Examples of the labelling substances used to label secondary and tertiary antibodies include enzymes, radioactive substances, fluorescent substances and other binding substances such as gold colloid, and magnetic powders. They may be bound to antibodies by known methods [Ishikawa, E. et al. Ann. New York Acad. Sci., 420, 74 - 89 (1983)].

Examples of enzymes which are actually used as labels are alkalline phosphatase, peroxidase, beta-KD-galactosidase; radioactive substances such as $^{125}I$, $^{131}I$, $^{14}C$, $^{3}H$ and fluorescent substances such as fluorescein isothiocyanate and tetramethyl Rhodamine isothiocyanate. These are merely illustrative, and any labeling substances heretofore used for immunological assaying methods may be used.

The secondary antibodies are generally used as solutions in solvents. For example, they may be used in the form of aqueous solutions containing buffers showing a pH of nearly neutrality in aqueous solution such as phosphate, Tris and Hepes and aqueous solutions containing nonionic surface active agents such as Triton and Tween.

The assay of human TF active substance in urine by the sandwich method using the primary and secondary antibodies described above, for example, by the method described in Wisdon, G. G. et al., Clin. Chem., 22, 1243 - 1255 (1978). One embodiment of this method will be specifically described.

An anti-human TF active substance monoclonal antibody (primary antibody) is fixed to a suitable insoluble carrier (such as a plastic container) to be referred to as a "fixed antibody". Then to avoid a non-specific binding between an insoluble carrier and a reagent or an assay sample to be measured, the surface of the insoluble carrier is coated with a suitable substances (such as bovine serum albumin; BSA).

The so obtained insoluble carrier to which the primary antibody is fixed is contacted with an assay sample (human urine or its dilution) for a fixed period of time at a fixed temperature to allow them to react. During this time, the human TF active substance in the assay sample binds to the fixed antibody (primary antibody). Then, the fixed antibody is washed with a suitable washing solution, then a solution (such as an aqueous solution) of an anti-human TF active substance monoclonal antibody (secondary antibody) labelled with a suitable labelling substance as contacted at a fixed temperature for a fixed time with a human TF active substance binding to the fixed carrier in the insoluble carrier to react it with the secondary antibody. The reaction product was washed with a suitable washing solution. Then, the amount of the labelling substance labelled to the secondary antibody on the insoluble carrier was determined by a customary method according to the labelling substance.

Thus, by intrapolating the measured value with respect to the standard curve, the amount of a human TF active substance in the assay sample may be calculated.

The assay reagent in the sandwich method described above is composed mainly of two types anti-human TF monoclonal antibodies which recognize different epitopes, one antibody being used as a primary antibody bound to an insoluble carrier and the other as a secondary antibody.

To utilize this reagent efficiently and conveniently, various suxiliary agents in addition to antibodies may be included to form a kit. Examples of such auxiliary agents include dissolving agents for dissolving solid reagents), washing agents to be used to wash the insoluble carriers, a substrate for measuring enzyme activity, and a reaction stopper therefor, which are normally used in the kit of an immunological assaying reagent.

Method B (competitive method)

Assaying of the TF active substance in urine by the competitive method may be carried out by using a labelled human TF active substance and an anti-human TF active substance monoclonal antibody fixed to an insoluble carrier.

The human TF active substance used in the competitive method binds to an anti-human TF active substasnce monoclonal antibody fixed to an insoluble carrier and preferably has an equivalent bindability and binding strength. It may be a TF isolated from human placenta or TF isolated from human brain each having a known structure, or TF extracted from the tissues. A preferred example may be a human TF active substance purified and isolated from human urine by a method to be described.

Labelling of the human TF active substance may be carried out by the same method as described in method A.

An example of the anti-human TF active substance monoclonal antibody fixed to an insoluble carrier may be the fixed antibody described with regard to the method A.

If a labelled human TF active substance monoclonal antibody and an anti-human TF active substance monoclonal antibody fixed (fixed antibody) is added to an assay sample (human urine or its dilution), the human TF active substance (a) in the assay sample, and the labelled human TF active substasnce (b) reacts competitively with the fixed antibody, and the human TF active substances (a) and (b) bind to the human TF active substances (a) and (b) according to their existing proportions with respect to the fixed antibodies.

After the reaction is carried out at a fixed temperature for a fixed period of time, the fixed antibody is separated from the reaction system, and the amount of the labelling substasnce bound to the fixed antibody is measured. By interpolating the amount with respect to the standard curve, the amount of the human TF active substance can be determined .

## Method C

In this method, a known amount (in an amount in excess of the reaction equivalent to human TF active substance which is anticipated to exist in an assay sample) of a labelled anti-human TF active substance monoclonal antibody (to be referred to as the labelled antibody) is added to the assay sample (human urine or its diluted solution) and reacted with a human TF active substance that can exist in the assay sample at a predetermined temperature for a fixed period of time. The labelled anti-human TF active substance monoclonal antibody (labelled antibody) used here may be those exemplified as the secondary antibodies mentioned in method A.

Then, an insoluble carrier to which a human TF active substance capable of binding to the labelled antibody (to be referred to as the fixed antigen) is added, and reacted at a predetermined temperature for a predetermined period of time to bind the unreacted labelled antibody to the fixed antigen. Then, the fixed antigen is separated from the reaction system, and the amount of the labelling substance bound to the fixed antigen is measured in accordance with a customary method.

By interpolating the measured amount with respect to the standard curve, the amount of the labelled antibody bound to the fixed antigen be determined. From this amount and the amount of the labelled antibody added first, the amount of the human TF active substance in the assay sample, the amount of the human TF active substance can be determined.

The fixed antigen used in the above method binds to the labelled antibody, and has equivalent antigenicity to the human TF active substance, but preferably may be a human TF active substance purified and isolated from human urine by the method to be described. The method of fixing the antigen may be the same physical or chemical method as described in regard to method A. According to the methods of this invention, a human TF active substance in human urine can be determined directly within a short period of time.

On the other hand, as stated hereinabove, the kidney tisssues incessantly perform metabolism and excretion, By some disease, damage is imparted to the tissue, and the human TF active substance separates from the surface of the cells, and is considered to be afloat in the urine. Accordingly, the human TF active substance in human urine can be a marker for diagnosis of kidney diseases such as renal failure, nephrosis syndrome and nephritis. In fact, when the present inventiors measured the amount of the human TF active substance in the urine of a hepatic patient by the method of this invention, its amount is much larger than in the amount in a healthy person (see Example 7).

Accordingly, the method of this invention is effective for the diagnosis of kidney diseases, particularly, renal failure, nephrosis syndrome, and nephritis.

In accordance with the method of this invention, the method of detecting the TF active substance in the human urine by the sandwich method will be specifically described as follows:

An insoluble carrier such as a plate or a bead having adsorbed an anti-human TF active substance monoclonal antibody (primary antibody) adsorbed thereto is blocked with a blocking reagent. Examples of the blocking reagent that may be used include bovineserum albumin (BSA), albumins of other animals, gelatin and skim milk.

Then, for the preparation of a calibration curve, human tissue factor apoprotein was diluted in various concentrations, and added. Further, an assay sample (human urine or its diluted solution) was diluted to suitable concentrations and added. After an immunological reaction is performed at room temperature for a certain period of time, and the insoluble carrier is washed with a solution containing Tween 20. Now, another peroxidase-labelled anti-hman TF active substance monoclonal antibody (secondary antibody) was added, and the reaction was carried out at room temperature for a predetermined pediod of time, followed by washing the reaction system. The reaction mixture was addded to the substrate solution to induce color formation. Each assay sample was determined for its absorbance at 415 nm. From a calibration curve prepared by using a human tissue factor apoprotein derived from human placenta, the content of the human TF active substance in the human urine is determined. The amount of the human TF active substance in the human urine is expressed as the amount (micrograms/ml of the urine) or the amount in the human urine collected per 24 hours (mg/day). If the amount of the human TF active substance is at least 5 micrograms/ml of urine, preferably at least 10 micrograms/ml, the subject is diagnozed as being suffering from a kidney disease (for example, nephritis). If the amount in the human urine collected for 24 hours (1 day) is at least 5 mg/day, preferably at least 10 mg/day, the subject is diagnozed as suffering from a kidney disease (for example, nephritis).

Now, the process of producing an anti-human tissue factor active substance monoclonal antibody used in the method of this invention will be described. If the anti-human TF active substance monoclonal antibody used in the method of this invention has the above-mentioned characteristics, the method of its production is not particularly limited, and it may be produced by a general method. A specific method is to immunize mouse spleen cells with a human tissue factor as an antigen, and fuse the these mouse spleen cells with mouse myeloman cells, and produce the monoclonal antibody from the resulting hybridoma cells [Köhler & Milstein: Nature, 256, 496 - 497 (1975)]

(1) Antigen

Examples of the antigen include a human placenta-derived tissue factor, human placenta-derived tissue facor apoprotein, a fragment on the N-terminus side not containing a domain binding to a phospholipid which fragment is obtained by decomposing a human placenta-derived TF apoprotein with CNBr, and the human TF apoprotein fragment represented by formula (1). Preferred are human placenta-derived TF apoprotein, the fragment on the amino group terminus side not containing a domain binding to a phospholipid obtained by decomposing TF apoprotein derived from human placenta with CNBr, and the fragment of human TF apoprotein which is represented by formula (1). Especially preferred are the fragment on N-terminus side not containing a domain binding to a phospholipid which is obtained by decompositng a human placenta-derived TF apoprotein with CNBr and the fragment of the human TF apoprotein which is represented by formula (1).

The human placenta-derived TF apoprotein used as the antigen is isolated from human placenta and purfified by the method of Gonmori et al. (Gonmori H. et al: Thromb. Haemostas. 36 , 90-103 (1976). Its amino acid sequence is represented by the following formula (III).

```
VPKPEWELITKFNTSKWTLNYAAVTNTTGS-NH2
  N
  Q
  V
  Y
  T
    VQISTKSGDWKSKCFYTTDTECDLTDEIVKDVK
                                    Q
                                    T
                                    Y
                                    L
                                    A
    TELYPTFEPSNEYLPEGASGTSEVNGAPYSFVR
  N
  L
  G
  Q
  P
      TIQSFEQVGTKVNVTVEDERTLVRRNNTFLSLR
                                     D
                                     V
                                     F
                                     G
                                     K
    EGKDVDILFENTNTKATKKGSSSSKWYYLTYILD
  N
  C
  F
  S
      VQAVIPSRTVNRKSTDSPVECMGQEKGEFREI
                                     F
                                     Y
                                     I
                                     I
                                     G
    NEKWSQGVGAKRCKHLSIALIIVLIIVVFVVA
  S
  P
  L
  N
  V
  S-COOH                          ... (III)
```

(3) Immunization of mice with the above antigen

Female BALB/C mice may be used, but mice of other strains may also be used. At this time, the immunization plan and the concentration of the antigen should be selected such that lymphocytes which have been stimulated in sufficient amounts of concentration may be formed. For example, the mouse is immunized three times peritoneally witth 50 micrograms at intervals of two weeks, and then 30 micrograms of the antigen was administered to the vein. Several days after the final immunization, the spleen cells were taken out for fusion.

(3) Cell fusion

9

The mouse spleen immunized as above was taken out aseptically, and a single cell suspension was prepared from it. These spleen cells were subjected to cell fusion from mouse myeloma single cells from a suitable line in the presence of a suitable fusion promotor. The preferred ratio of the spleen cells to the myeloma cells is about 20:1 to about 2:1. The use of 0.5 to 1.5 ml of a fusion medium per about $10^8$ spleen cells is suitable.

The mouse myeloma cells used for cell fusion are well known, and P3-X63-Ag8-U1 cells (P3-U1) [Yelton D.F. et al.; Current Topics in Microbiology and Immunology, 81, 1 (1978)] are preferred.

A preferred fusion promotor, for example, polyethylene glycol having an average molecular weight of 1000 to 4000 may advantageously be used. Other fusion promotors known in this field may also be used.

(4) Selection of Fused Cells

In a separate receptacle (e.g., a microtiter plate), a mixture of Unsufed spleen cells, unfused mouse myeloma cells and hybridoma cells were diluted with a selective medium which does not support unfused mouse myeloma cells, and cultivated for a time period sufficient to cause the unfused cells to die away (for about 1 week). The medium, for example, HAT medium which does not support the unfused mouse myeloma cells is used. In the selective medium, the unfused myeloma cells die away. Since the unfused spleen cells are non-tumorous cells, they die away after a certain period of time (after 1 week). On the other hand, the fused cells have both the tumorous nature of the myeloma cells and the nature of the spleen cells they can survive in the selective medium.

(5) Recognition of anti-human tissue factor antibody in each receptacles:

Thus, after the hybridoma cells are detected, the supernant liquid was collected. Antibodies to human tissue factors were screened by the enzyme linked immuno-sorbent assay (ELISA).

(6) Cloning of hybridoma cells producing the desired antibodies

When the hybridoma cells producing the desired antibody are cloned by a suitable method (for example, the limiting dilution method) the antibodies are produced by two different methods. According to a first method, by cultivating the hybridoma cells in a suitable medium for a certain predetermined period of time, the monoclonal antibodies produced by the hybridoma cells can be obtained from the supernatant liquid. According to a second method, the hybridoma cells can be injected intraperitoneally to syngeneic or semi-syngeneic mice. after a certain period of time, the monoclonal antibodies produced by the hybridoma cells can be obtained from the blood and the ascites of a host animal.

The monoclonal antibody EX6 may be produced by the hybridoma cells obtained from spleen cells obtained by immunizing human placenta-derived TF apoproteins of formula (III). On the other hand, the aforesaid monoclonal antibodies GX3 and GX4 cannot be obtained by immunization with the TF apoprotein of formula (III), but can be produced by hybridoma cells from the spleen cells obtained by treating TF apoprotein of formula (III) with CNBr.

If the anti-human tissue factor active substance monoclonal antibodies produced as above are used, the human tissue factor active substance can be separated and purified from a liquid containing human tissue factor active substances such as human urine.

Thus, according to another aspect of this invention, there is provided a method of purifying a human tissue factor active substance, which comprises bringing an anti-human tissue factor active substance monoclonal antibody bound to an insoluble carrier into contact with a liquid containing a human tissue factor substance, separating the insoluble carrier to which the antibody is fixed from the said carrier, and subjecting the human tissue factor active substacne to an elution treatment.

The insoluble carrier used in this method to bind the anti-human TF active substance monoclonal antibody is not particularly limited if it is a substantially insoluble solid used for an eluent for elution treatment, and various materials may be used. Examples include Sepharose, agarose, a polyacrylamide resin, cellulose and its derivatives, dextran, and maleic acid polymer. These materials are usually in the form of beads, fibers, powders or gels.

Examples of the method of binding an antibody to these materials may include the method of forming an amide linkage between the antibody and the the carboxyl groups or amino groups that can exist in these materials; and the method of introducing functional groups capable of reacting with these antibodies, for example epoxy groups thiopropyl groups and CNBr groups into these materials, and binding the antibodies to these groups (see, for example, Axen, R et al. Nature, 214, 1302-1304 (1967)].

The insoluble carrier to which the anti-human TF active substance monoclonal antibody is bound may be, for example, filled in a column. By flowing a liquid containing a human TF active substance into the column, the liquid is brought into contact with the insoluble carrier. Then, in accordance with an ordinary melting treatment method, an eluting agent is poured into the column, fractions showing human TF activity are gathered from the eluent. By recovering the human TF active substance from the fraction by a method, [for example George J. Broze et al, J. Biochem. Chem., 260, 10917 - 10920 (1985)], a substantially pure human TF active substance can be obtained.

Examples of the eluent that can be used in the eluting treatment include, for example, Glycine-HCl (pH 2.5), 3M NaSCN (pH 7.4), 2.5M NaI (pH 7.5), 6M guanidine-HCl (pH 3.1), 8M urea (pH 7.0) and 50%, v/v, ethylene glycol (pH 11.5).

Of these solutions containing kaotropic ions, such as 3M NaSCN pH 7.0 solution, are preferred.

The human tissue factor active substance which can be separated from human urine by the above purifying method is a novel substance which amino acid sequence is partly differs from the tissue factors reported in the previous publications.

The human TF active substance purified from human urinedevelops blood coagulation activity by activating factor VII in the presence of a phospholipid and a calcium ion, has an optimum pH of 7.5 to 8.5 and can develop activity in a pH range of 5.5 to 12. It has a molecular weight of 54,000 to 60,000, and is further a TF-like protein which is decomposed with cyanogen bromide into two fragments having a molecular weight of 36,000 to 40,000 and 18,000 to 20,000.

When a portion having a molecular weight of about 36,000 obtained by decomposition with CNBr is further reductively alkylated, it further separate into a fragment having a molecular weight of about 12,000 and a fragment having a molecular weight of about 24,000. When the fragment having a molecular weight of about 12,000 is further analyzed in amino acid sequence, it is found that from the N-terminus side, it has the following sequence:

| 1 | 2 | 3 | 4 | 5 | 6 | 7 | 8 | 9 | 10 | 11 |
|---|---|---|---|---|---|---|---|---|----|----|

Lys-Glu-Gly-Ala-Ala-Ile-Leu-Phe-Glu-Pro-Thr

Furthermore, this human urine-derived TF-like protein can be likewise recognized by monoclonal antibody GX3 which can recognize TF derived from human placenta.

Furthermore, when the TF-like protein is stirred at room temperature for 10 minutes with respect to physiological saline and maintained stationary for 1 hour, this protein showed a solubility of about 50 mg/100 ml of water.

The physicochemical properties of the TF active substance derived from human urine showing these properties are as follows:-

| (1) | Amino acid composition | Content (wt. %) |
|---|---|---|
| | Asp (and/or Asn) or | 10 - 11 |
| | Gln (and/or Glu) | 8 - 9 |
| | Ser | 10 - 11 |
| | Gly | 9 - 10 |
| | His | 2 - 3 |
| | Arg | 5 - 6 |
| | Thr | 8 - 9 |
| | Ala | 7 - 8 |
| | Pro | 6 - 7 |
| | Tyr | 4 - 5 |
| | Val | 7 - 8 |
| | Met | 1 - 2 |
| | Ile | 2 - 3 |
| | Leu | 8 - 9 |
| | Phe | 3 - 4 |
| | Lys | 3 - 4 |
| | Trp | (detection impossible) |
| | Cys | (detection impossible) |

The amino acid composition was analyzed by hydrolyzing the urine TF active substance at 110 °C for 24 hours in a vapor of 6N-HCl containing 0.1 % (v/v) of phenol, and by using an amino acid automatic analyzing device (PICO-TAG; made by Waters Company.

(2) Carbohydrate content       25 to 30 % by weight

(3) isoelectric point       6.8 - 7.2

(4) Ultraviolet absorption spectrum: $\lambda_{max}278$ nm

(5) Specific activity (blood coagulating activity)

showed an activity about 1/8 to 1/10 of the activity of human placenta-derived TF which was purified by the method described in Thromb. Haemostas. 36 , 90 - 103 (1976).

The human urine-derived TF active substance having such characteristics is considered to have utility as a hemostatic for trauna and after surgical operation, as a therapeutic agent for hemophilia patients having a neutral antibody to factor VIII, and as an initiator for blood coagulation.

BRIEF DESCRIPTION OF THE DRAWINGS

Figuire 1 shows the strength of binding of the monoclonal antibody of this invention to a human placenta TF active substance;

Figure 2 shows the differences in the antigen recognizing sites (epitopes) of three types of the monoclonal antibodies of this invention;

Figure 3 shows effects of the monoclonal antibodies of this invention on the activities of tissue factor;

Figure 4 is a calibration curve for assaying human TF active substances;

Figure 5 is a summary of the relation of various kidney deseases to contents of human TF active substances in human urine;

Figure 6 shows the relation between the activities of urine TF active substance and the pH; and

Figure 7 shows the outside of the overall composition of the protein of this invention.

Examples

In the following, the present invention will be described by citing Examples.

Example 1 Obtaining hybridoma (fused cells) producing a monoclonal antibody binding to a human TF active substance

A human TF apoprotein purified from a human placenta extract (to be referred to as antigen 1) and a fragment on the N-terminus side not containing a domain binding to a phospholipid (to be abbreviated as antigen 2) obtained by decomposing human placenta-derived TF apoprotein with CNBr were each immunized with (four weeks of age) of female Balb/C mice (total three mice) four times at intervals of 14 days. In the first immunization, 50 micrograms of the antigen was dissolved in physiological saline and the solution was mixed with an equal amount of Freund complete adjuvant, and the emulsion was administered peritoneally. In the 2nd and third immunization, 50 micrograms of the antigen was mixed with Freund incomplete adjuvant, and administered intraperitoneally. In the final immunization (fourth immunization), 30 micrograms of the antigen was additionally administered through the mice. Three days after the final immunization, the spleen cells of the immunized mice were used for cell fusion.

The extract spine cells of the mice and the myeloma cells (P3U1) of the same strain of mouse were mixed at a rate of about 5:1, and subjected to cell fusion by using 50% polyethylene glycol 1540 by the method of Köhler and Milstein.

The cells after fusion were suspended in RPMI 1640 containing 10% of fetal serum so that the cell concentration became $1 \times 10^5$ cells/ml, and poured onto a 96-well microplate at a rate of 100 microliters per well.

The hybridomas (fused cells ) were cultivated in a $CO_2$ incubator (5 % $CO_2$ 37 °C). The culture medium was exchanged by using HAT medium containing hypoxanthine, aminoputerine and thymidine (HAT medium), and the cells were proliferated in the HAT medium. Hydridomas composed of the spleen cells and the mouse myeloma cells were screened.

The antibody in the culture supernatant in the hybridoma reacted to a microliter plate to which tissue factor apoprotein purified from human placenta was adsorbed, and the detection was performed by the ELISA method. Among the total 1022 wells on which the hybridoma were seeded formation of colonies was seen in 669 wells. Of these the number of wells which showed antibody production positive showing bindability to human placenta-derived tissue factor apoprotein was 356 wells. (Table 1)

## Table 1: cell fusion

| Cell fusion ╲ Immunizing antigen | Antigen 1 | Antigen 2 |
|---|---|---|
| (S) Spleen cells | $1.63 \times 10^8$ | $0.88 \times 10^8$ |
| (P) Myeloma cells | $3.36 \times 10^7$ | $2.58 \times 10^7$ |
| S/P ratio | 4.9 | 3.4 |
| Hybridoma (cells/well) | $1.2 \times 10^5$ | $6.5 \times 10^4$ |
| (A) colony formation (well)(percent formation %) | 162 (58.3) | 285 (79.2) |
| (B) Antigen binding positive well (B/A x 100%) | 63 (38.9) | 253 (88.8) |

Four wells among these antibody acid-positive wells were repeatedly cloned two times by the limiting dilution method, and three monoclones were obtained. The resulting clones were suspended in a 90 % bovine serum solution containing 10 % of DMSO, and stored in liquid nitrogen. The monoclonal antibody produced by each clone was proliferated in intraperitoneally in Balb/C mice, and the clone was purified from the ascites by using a column of Protein A/Sepharose 4B.

Example 2
Classes of purified monoclonal antibodies

The class of IgG of each clone purified from mouise ascites was determined by the Ouchterlony method.

## Table 2
## Classes of monoclonal antibodies

| Antigen | Antibody | H chains | L chains |
|---|---|---|---|
| Antigen 1 | EX 6 | $\gamma_{2a}$ | $\kappa$ |
| Antigen 2 | GX 3 | $\gamma_{2a}$ | $\kappa$ |
| Antigen 2 | GX 4 | $\gamma_{2a}$ | $\kappa$ |

Measurement of dissociation constants (KD)

The purified monoclonal antibodies (GX3, GX4 and EX6) were used each in Immunobeads (made by Bio-Rad Co.) and labelled with $^{125}$I to prepare antibody solutions.

To a 96-well-microtiter plate (made by FLOW LAB Co., Titertek), purified urinary TF active substance, and placenta TF active substance were added each in a concentration of 1 micrograms/ml at a rate of 50 microliters/well to perform adsorption overnight at 4 °C. After removing the antigenitc solution, 10 mM phosphate buffer (pH 7.2) containing 1 % BSA and 0.125 M NaCl was added at a rate of 100 microliters/well, and allowed to stand at room temperature for 2 hours. After removing the BSA solution, $^{125}$I-labelled monoclonal antibody was added to the wells of the plate so as to have various concentrations in the range of 0.1 to 5 micrograms/ml, and reacted at 37 °C for 2 hours. Then the wells were washed three times by adding 10 mM phosphate buffer (pH 7.2) containing 0.05 % Tween 20 at a rate of 100 microliters/well.

The wells were cut out from the plate, put into a plastic test tube, and $^{125}$I radioactivity was measured by a gamma counter (radioactivity bound to the solid antigen (bound (cpm)). At the same time, the radioactivity of the monoclonal antibody solution for addition to the wells was measured at the same time (the total radioactivity of the added antibody: total (cpm)).

By plotting the concentration of the monoclonal antibody added on the axis of abscissas, and the ratio of total-bound (the radioactivity not bound to the solid antigen: free (cpm)) to the Bound on the axis of ordinates, and the dissociation constant (KD) was calculated from the Scachard plot.

## Table 3: Dissociation constant

| Antibody | Antigen | |
| --- | --- | --- |
| | Placenta TF active substance | Urine TF active active substance |
| GX3 | $2.26 \times 10^{-9}$ M | $1.07 \times 10^{-9}$ M |
| GX4 | $1.14 \times 10^{-9}$ M | $1.02 \times 10^{-9}$ M |
| EX6 | $1.08 \times 10^{-9}$ M | $1.35 \times 10^{-9}$ M |

Example 3

Bindability to human placenta-derived TF apoprotein

Human TF apoprotein in a concentration of 5 micrograms/ml extracted and purified from human placenta was caused to be adsorbed to a microtiter plate and after blocking with 1% BSA, was reacted with a monoclonal antibody solution 0.16 - 5.0 micrograms/ml) diluted to a suitable concentration. Then, an anti-mouse antibody labelled with alkaline phosphatase was added

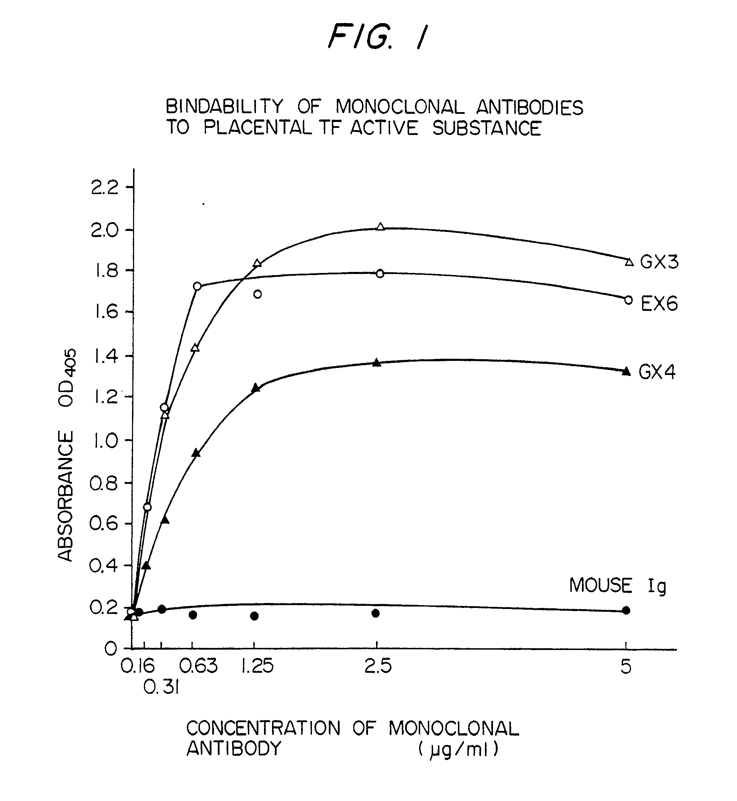

The resulting three types of monoclonal antibodies showed strong bindability to human TF apoprotein (Figure 1),

Example 4 Differences of the epitope recognition sites in monoclonal antibody

Human placenta-derived TF apoprotein was adsorbed to a microtiter plate in a concentration of 5 micrograms/ml, and after blocking with 1% BSA, each of various monoclonal antibody solutions (0.16 to 5.0 micrograms/ml) and a EX6 antibody labelled with peroxidase were simultaneously reacted with the antigen.

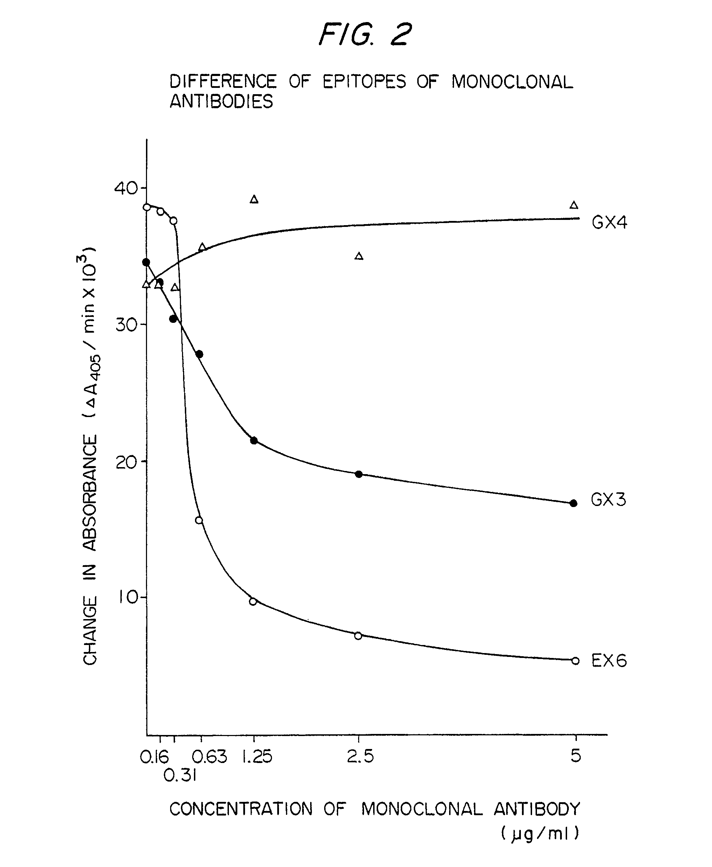

The results suggested that Ex6 and GX4 can simultaneously bind to human TF apoprotein and the epitopes are different. There is competitive inhibition in the binding of EX6 and GX3 to human TF apoprotein, Since the inhibition of the binding of EX6 is about 50% of the inhibition of binding to the

EP 0 417 298 A1

peroxidase-labelled EX6, it is suggested that the epitopes of EX6 and GX3 are different and are sterically approximate sites (Figure 2).

Example 5

<u>Bindability of the monoclonal antibody to human TF apoprotein treated with CNBr</u>

HCOOH was added to 40 micrograms of human TF apoprotein to form a 70% HCOOH solution. CNBr powder was added to this solution and dissolved, and the reaction was performed at room temperature for 18 hours. Thereafter, HCOOH was dried up. The protein was dissolved by adding 40 microliters of $H_2O$, and then an amount equivalent to 2 micrograms was reduced in the presence of 2-mercaptoethanol. By using a gradient gel having a concentration of 4 to 20 %, SDS-polyacrylamide electrophoresis was carried out (fragments having a molecular weigth of 31,000 and 27,000). For comparison, A TF apoprotein (M.W. 58,000) not treated with CNBr was similarly reduced, and electrophoresis was carried out.

After electrophoresis, the protein in the gel was electrically transferred to a nitrocellulose membrane by using a blotting device. The nitrocellulose membrane was blocked with TBS containing 3 % of gelatin (20 mM Tris solution-0.15M NaCl, pH 7.4), and a 1% gelatin-TBS solution containing each of various monoclonal antibodies (GX3, GX4 and EX6) in a concentration of 2 micrograms/ml was reacted overnight at room temperature with the nitrocellulose membrane. The nitrocellulose membrane was washed with 0.05 % of Tween 20-TBS three times, and the reaction was carried out with a 1% gelatin-TBS solution of a peroxidase-labelled anti-mouse Ig antibody at room temperature for 4 hours at room temperature. After washing, a 4-chloro-1-naphthal substrate solution was added to color the enzyme-labelled antibody bound to the nitrocellulose membrane The protein bound to the monoclonal antibody could be detected as a deep blue band.

The three monoclonal antibodies obtained had the binding properties given in Table 4.

16

## Table 4

| To be bound to | Monoclonal antibody | | |
|---|---|---|---|
| | GX3 | GX4 | EX6 |
| Human placenta-derived TF apoprotein having a phospholipid binding thereto (re-constituted in vitro) | △～○ | ○ | X |
| Human placenta-derived TF apoprotein (M.W. 58,000) | ○ | ○ | ○ |
| An N-terminus fragment not containing a domain having a phospholipid of the apoprotein not binding thereto which results from treatment with CNBr (M. W. 31,000) | ○ | ○ | X |
| A C-terminus fragment containing a domain binding to a phospho-lipid of the above apoprotein which resulted from treatment with CNBr (M. W. 27,000) | X | X | ○ |
| Reduced apoprotein | ○ | X ～△ | ○ |

○: Strongly binding

△: Weakly binding

X: Not binding

Example 6
Effects of monoclonal antibodies on the activity of tissue factor

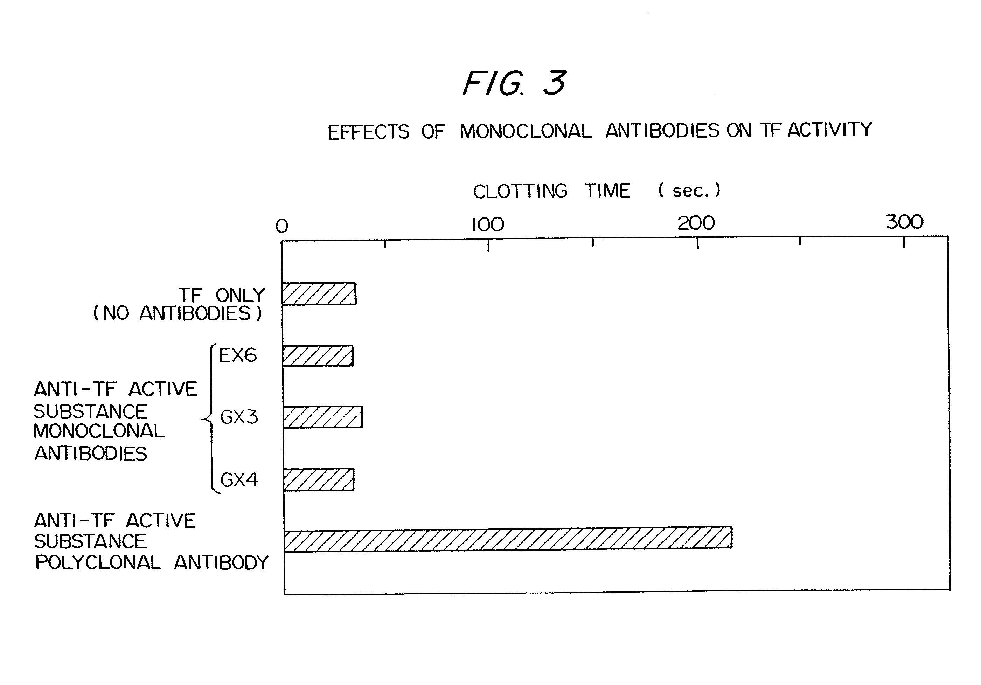

50 micrograms of each of the resulting monoclonal antibodies (GX3, GX4, EX6) was mixed with 100 micrograms of human placenta-derived tissue factor to form 1 ml of a solution, and reacted at 37 °C for 1 hour, and allowed to stand overnight at 4 °C. Out of this solution, a 200 microliter portion was added to 100 microliters of human plasma, and the coagulation time (PT) was measured. As a control, a rabbit anti-serum to TF apoprotein was used instead of the monoclonal antibody. The measurement was made by using blood coagulation analyzer CA-100. Figure 3 shows the coagulation times of the samples in a graph.

The monoclonal antibodies (GX3, GX4 and EX6) did not affect the activity of inducing coagulation of human placenta-derived TF.

Example 7
Detection of a human TF active substance in an assay solution

Monoclonal antibody GX3 was diluted with PBS (10 mM phosphate buffer - 0.15M NaCl pH7.4) so as to provide a concentration of 20 micrograms/ml, and 100 microliters of the resulting solution was added to a well of a microtiter plate. It was allowed to stand overnight to induce adsorption of the antibody to the solid phase. PBS containing 1% BSA was added at a rate of 150 microliters/well. Successively, the plate was allowed to stand at room temperature and allowed to stand for 2 hours. The plate was washed with PBS (washing buffer (containing 0.05% Tween 20 and 0.1% BSA. Then, human placenta-derived TF apoprotein was diluted with the washing buffer in a concentration of 25 ng/ml, 50 ng/ml and 100 ng/ml, and human urine was diluted to 80-fold and 40-fold and the diluted solutions were added in an amount of 100 microliters/well and reacted at 37 °C for 1 hour. The plate was washed with the washing buffer three times. Then, the peroxidase-labelled monoclonal antibody GX4 was diluted to a concentration of 300 ng/ml, and added in an amount of 100 microliters/well, and reacted at 37 °C for 1 hour. After washing with the washing buffer three times, and the substrate solution (ABTS) in an amount of 100 microliters/well, and the absorbance at a wavelength of 415 nm was measured.

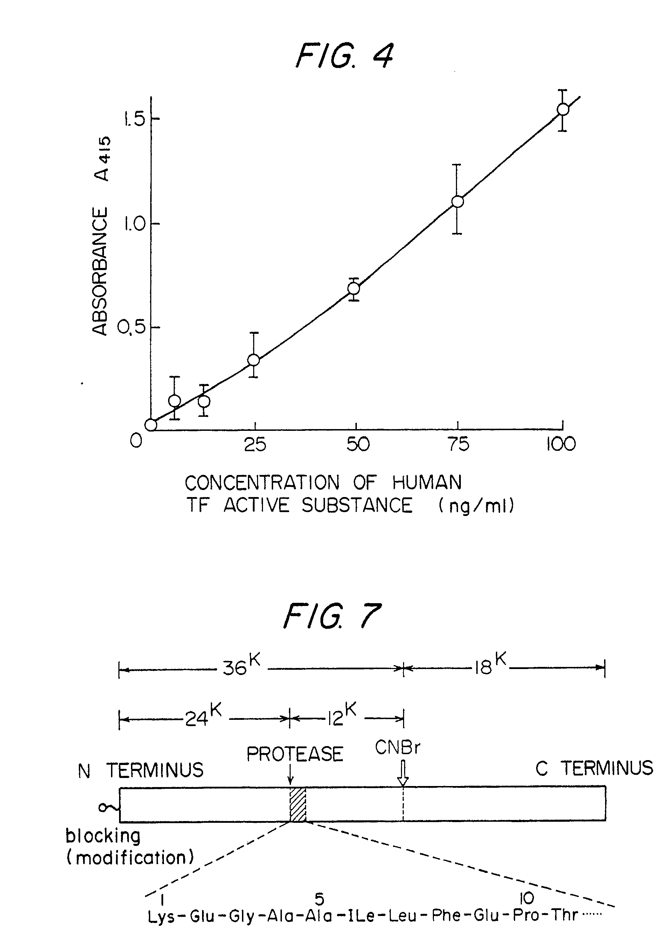

Figure 4 shows a calibration curve prepared by using human placenta-derived tissue factor apoprotein. The concentration of the human TF (the axis of abscissas) and the absorbance (the axis of ordinates) are in a linear relation, and by using this callibration curve, a human TF active substance in a solution (human urine) can be determined.

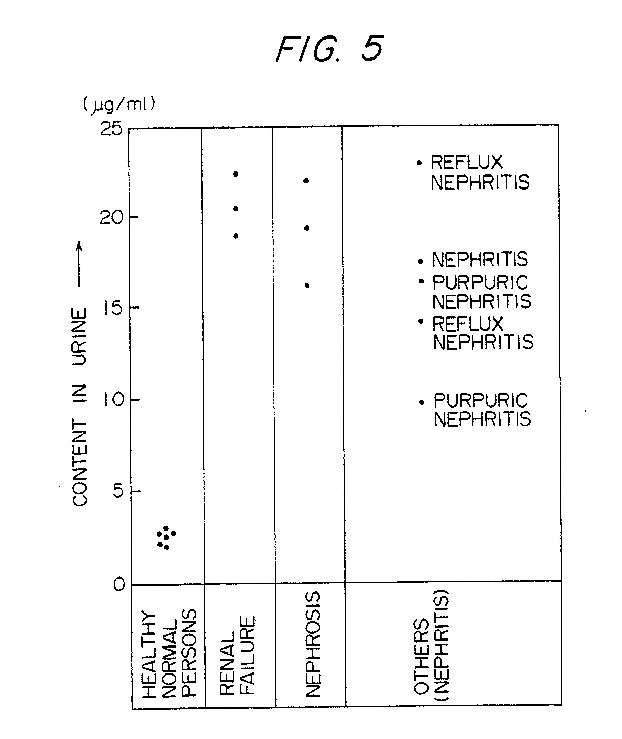

Figure 5 summarizes the results of measurements. The human urine in a healthy person contained human TF in a concentration of 2 to 3 micrograms/ml. On the other hand, in human urine of patients with renal failure, nephrosis syndrome and various conditions of nephritis, TF was defected in a much higher concentration than in normal healthy persons as 10 to 25 micrograms/ml.

Example 8
Purification of TF active substance from urine

500 ml of urine of a normal healthy person containing 20 units/ml of transyrol protease inhibitor) was filtered by filter paper (Toyo Filter Paper No. 2), and the passed through an anti-TF monoclonal antibody column (column volume: 10 ml; the amount of the antibody bound: 8 mg) at a flow rate of 15 ml/hr. The column was washed with 200 ml of a washing buffer A (containing 20 mM Tris-HCl, 0.5M NaCl pH 7.6, 0.05% Tween 20 and 20 units/ml of transyrol), and then with 150 ml of a washing buffer (20 mM Tris-HCl, 0.10 M NaCl, pH 7.6, and 20 units/ml of transyrol. It was confirmed that the washing fraction had an absorbance ($A_{280}$) of 0 at a wavelength of 280 nm. Subsequently, 3M NaSCN solution (pH 7.0) was passed through the column at a flow rate of 20 ml/hr, and the TF active substance in the urine which bound to the antibody column was eluted. Immediately then, the NaSCN salt was removed by dialysis, and by using a protein assay reagent(dye reagent made by Bio-Rad Co., Ltd.), the amount of protein determined by colorimetry. It was 591 micrograms. The column was washed by 6M guanidine-HCl (pH 3.1), and preserved by passing 200 ml of the washing buffer B. The above purification operations excepting the measurement of the concentration of the protein were all performed at 4 °C.

Example 9
Properties of purified urine TF active sub stance

(1) Determination of molecular weight

The molecular weight of urine TF active substance was determined by the SDS-polyacrylamide electrophoresis method using a gel with a concentration of 10 to 20%. One microgram of protein was caused to flow in each lane, and dyed with Coomassie. The molecular weight was calculated from the mobility.

(2) Measurement of the blood coagulation (clotting) activity

Urine TF active substance (1.25 - 20 micrograms) was mixed with 20 micrograms of a phospholipid, and by adding a CaCl₂ solution was added to a final concentration of 2 mM, and the reaction was performed at 37 °C for 30 minutes. 200 microliters of this reaction solution was added to 100 microliters of plasma, and the coagulation time was measured (PT). The amount of protein micrograms added to the plasma in relation to the coagulation time (seconds) is shown in Table 5. As a result, the molecular weight of the urine TF active substance was 54,000 under reducing conditions and it was found to have the activity of inducing blood coagulation.

## Table 5

### Activity of TF active substance purified from urine

| Amount of protein added to blood (micrograms) | Blood coagulation time (seconds) | |
|---|---|---|
| | Urine TF active substance | Placenta TF protein |
| 1.25 | 332 | 237 |
| 2.5 | 334 | 43 |
| 5 | 192 | 31 |
| 10 | 151 | 36 |
| 20 | 139 | 32 |

Example 10

Influence of pH on the developmenmt of urine TF active substance

Human fresh plasma (500 microliters) was added 0.1M citrate buffer (pH 3.0), or 0.05M Tris buffer (pH 9.0) or 0.1M soium hydrogen carbonate-NaOH buffer (pH 12.5) to adjust the pH to various values. By using water purified by using MILL-Q (made by Millipore Co., Ltd.), the amout of the solution was adjusted to 550 microliters.

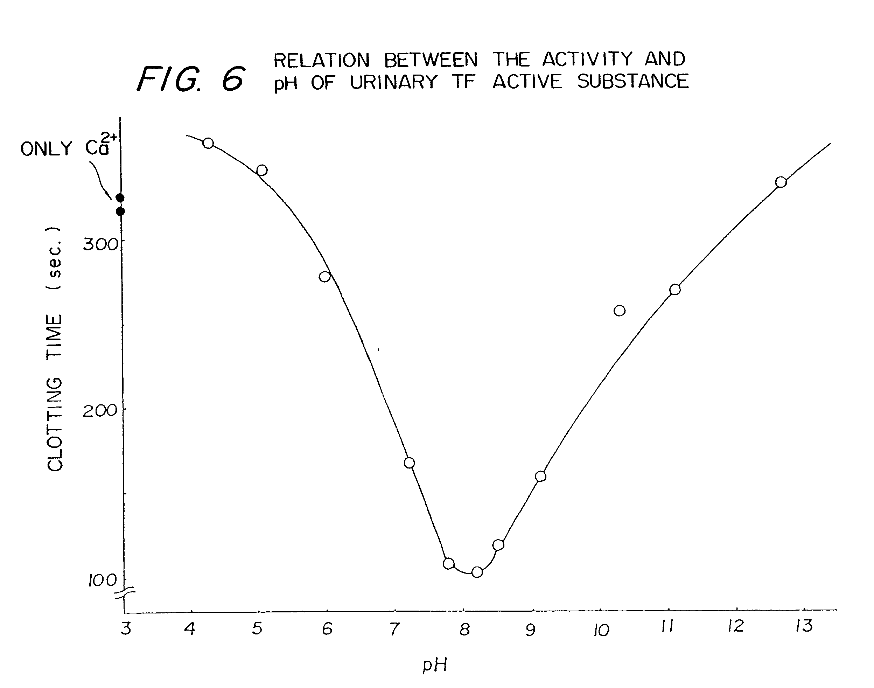

10 micrograms of urine TF active substance was mixed with 20 micrograms of phospholipid, and 0.1 M CaCl₂ solution was added to a final concentration of 2 mM, and reacted at 37 °C for 30 minutes. 200 microliters of the reaction solution was added to 100 microlirters of human plasma adjusted in pH, and the coagulation time (PT) was measured. The relation between the pH and the coagulation time was shown in Figure 6. Consequently, it was found that the optimum pH at which the urine TF active substance developed blood coagulation activity was 7.5 - 8.5, and the pH range in which the activity could be developed was 5.5 to 12.

Example 11 Analysis of the structure of the urine TF active substance

(1) Solubilization of urine TF active substance

CNBr was added to 100 micrograms of urine TF active substance (100 microliters of a 70% HCOOH solution), and reacted for 12 hours at room temperature (23 °C. After drying , 100 microliters of 0.2M phosphate buffer-0.15M NaCl pH 7.8 (containing 0.10% SDS, 5 % 2-mercarptoethanol). The mixture was heated for 3 minutes at 100 °C. The mixture was cooled to room temperature, and to the protein solution was added 5 units/ml of N-glycanase (Waters Co.). They were reacted at 37 °C for 2 days to solubilize the urine TF active substance. (2)

(2) Isolation of the fragment obtained by decomposition with CNBr

To 100 microliters of the solubilized solution the protein obtained in (1) was added 400 microliters of distilled pure water containing 0.1 % trifluoroacetic acid (TFA). After mixing, the mixture was passed through a $C_{18}$ column (Waters Co., 300 Å, inverse phase column. By using 0 to 60 % of acetonitrile concentration gradient, 39 micrograms of a fragment having a molecular weight 36,000 (36K) and 26 micrograms of a fragment having a molecular weight of 18,000 (18K) were isolated.

(3) N-terminal amino acid sequence of the 12K fragment obtained by digestion with protease

Protease (Berlinger Manheim Co., Ltd. , Asp-N) in an amount of 0.1 micrograms was added to an aqueous solution of the reductively alkylated 36K fragment (20 micrograms, and the reaction was carried ouit at 37 °c for 12 hours. Distilled water containing 0.1 % of TFA and 1 % of acetonitrile in equal amounts were added in equal amounts to the solution, and mixture. The mixture was passed throuigh phenyl-5PW column (TOSO Co., Ltd. to isolate fragments having molecular weihts of 12K, 24K and 36K. The N-terminusuamino acid sequence of the 1.8 micrograms (150 mol) of the 12K fragment was analyzed, by means of Protein Sequencer 470A (appllied Biosystems Co., Ltd. As a result, the following sequence was identified.

$$
\begin{array}{ccccccccccc}
1 & 2 & 3 & 4 & 5 & 6 & 7 & 8 & 9 & 10 & 11 \\
\text{Lys} & \text{Glu} & \text{Gly} & \text{Ala} & \text{Ala} & \text{Ile} & \text{Leu} & \text{Phe} & \text{Glu} & \text{Pro} & \text{Thr}
\end{array}
$$

On the other hand, the 24K fragment (2.2 micrograms; 92 pmol), and the 36K fragment (3.4 micrograms (94 pmol) were applied to the sequencer. But in each cycle, no amino acids were detected, and it was suggested that the amino acid at the N terminus was modified or blocked.

From the three amino acid sequence analysis of the three fragments, it can be thought that the sequence composed of the above 11 amino acids existed in the part shown in the schematic view in Figure 7.

With respect to the TF active substance isolated from human urine, the amino acid composition, the carbohydrate content, the specific activity, the isoelectric point and max value were analyzed. The results are summrized in Tables 6 and 7.

## Table 6

Analysis values of Urine
TF active substance

| Analysis of amino acid composition | |
|---|---|
| Amino acids | Content (%) |
| Asp(Asn) | 10.4 |
| Gln(Glu) | 8.6 |
| Ser | 10.5 |
| Gly | 9.5 |
| His | 2.7 |
| Arg | 5.7 |
| Thr | 8.4 |
| Ala | 7.7 |
| Pro | 6.0 |
| Tyr | 4.4 |
| Val | 7.2 |
| Met | 1.8 |
| ILe | 2.3 |
| Leu | 8.0 |
| Phe | 3.5 |
| Lys | 3.3 |
| Trp | N.D.(Not Detected) |
| Cys | N.D.(Not Detected) |
| Carbohydrate content   28.8% | |
| Isoelectric point      6.8-7.2 | |
| $\lambda$max      wave length 278nm | |

## Table 7: Comparison of TF activity

| Protein | Amount | Coagulation time (seconds) | Specific activity |
|---|---|---|---|
| Placenta TF apoprotein | 30 µg | 23 | 1.00 |
| Urine TF active substance | 30 µg | 185 | 0.12 |

Possibility of Industrial Utility

As stated in detail hereinabove, the use of this invention permits direct assaying of a human TF active substance. Even a solution of a human TF active substance (for example, in urine) can be determined within s short period of time quantitativelyu without undergoing influences by other foreign materials. Furthermore, by assaying a human TF active substance in human urine, a kidney disease can be diagnozed, and the degree of damage to the kidney tissue can be grasped.

## Claims

1. A method of detecting a human tissue factor active substance, which comprises immunologically assaying a human tissue factor active substance in human urine.

2. The method of claim 1 wherein one of two kinds of anti-human tissue factor active substasnce monoclonal antibodies which recognize different epitopes of the human tissue factor active substance is fixed to an insoluble carrier, and used as a primary antibody, and the other monoclonal antibody is used as a secondary antibody, and the human tissue factor active substance is measured by a sandwich method.

3. The method of claim 2 wherein the anti-human tissue factor active substance monoclonal antibody binds specifically to the human tissue factor active substance and does not inhibit the blood clotting activity of the human tissue factor active substance.

4. The method of claim 2 wherein the secondary antibody is labelled with an enzyme.

5. The method of claim 1, which comprises adding a labelled human tissue factor active substance and an anti-human tssue factor active substance monoclonal antibodyu fixed to an insoluble carrier to human urine, and assaying the human tissue factor active substance in the human urine by a competitive method.

6. The method described in claim 1, which comprises adding a known amount of an anti-human tissue factor active substance monoclonal antibody to human urine and reacting it with a human tissue factor active substance that can exist in the urine, then adding an insoluble carrier to which is fixed a human tissue factor active substance which can bind to the antibody, thereafter separating the insoluble carrier, and measuring the amount of the antibody binding to the insoluble carrier to defect the human tissue factor active substance in the human urine.

7. The method of claim 1 which is used for diagnosis of kidney diseases, particularly renal failure, nephrosis syndrome or nephritis.

8. An anti-human tissue factor active substance monoclonal antibody which binds to a human tissue factor active substance, and does not inhibit the blood coagulation activity of the human tissue factor active substance.

9. The monoclonal antibody described in claim 8 which has a dissociation constant, with respect to the human tissue factor active substance, of not more than $5 \times 10^{-9}$.

10. A hybridoma cell which produces the monoclonal antibody described in claim 6.

11. A method of purifying a human tissue factor active substance, which comprises bringing an anti-human tissue factor active substance monoclonal antibody found to an insoluble carrier in contact with a liquid containing a human tissue factor active substance, separating the insoluble carrier having the antibody fixed thereto from the liquid, and subjecting the human tissue factor active substance to an elution treatment.

12. The method of claim 11 wherein the liquid containing the human tissue factor active substance is human urine.

13. A substantially pure human tissue factor active substance whch can be separated from human urine.

## *FIG. 1*

BINDABILITY OF MONOCLONAL ANTIBODIES
TO PLACENTAL TF ACTIVE SUBSTANCE

FIG. 2

DIFFERENCE OF EPITOPES OF MONOCLONAL ANTIBODIES

## FIG. 3

### EFFECTS OF MONOCLONAL ANTIBODIES ON TF ACTIVITY

CLOTTING TIME ( sec. )

EP 0 417 298 A1

## FIG. 4

CONCENTRATION OF HUMAN
TF ACTIVE SUBSTANCE (ng/ml)

## FIG. 7

## *FIG. 5*

FIG. 6    RELATION BETWEEN THE ACTIVITY AND
          pH OF URINARY TF ACTIVE SUBSTANCE

EP 0 417 298 A1

# INTERNATIONAL SEARCH REPORT

International Application No PCT/JP90/00127

## I. CLASSIFICATION OF SUBJECT MATTER (if several classification symbols apply, indicate all) 6

According to International Patent Classification (IPC) or to both National Classification and IPC

Int. Cl⁵    G01N33/53, G01N33/577, C12P21/08,
            C07K1/14, C07K13/00, A61K37/02

## II. FIELDS SEARCHED

### Minimum Documentation Searched 7

| Classification System · | Classification Symbols |
|---|---|
| IPC | G01N33/53, G01N33/577, C12P21/08, C07K1/14, C07K13/00, A61K37/02 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| Y | JP, A, 63-301797 (Genetic Inc.), 8 December 1988 (08. 12. 88) & EP, A, 278776 & AU, A, 8811705 | 13 |
| Y | JP, A, 63-218625 (Genetic Inc.), 12 September 1988 (12. 09. 88) & EP, A, 266993 & ZA, A, 8708249 | 13 |
| Y | THROMBOSIS RESEARCH, Vol. 52, No. 3, (1988), [MONOCLONAL ANTIBODY ANALYSIS OF PURIFIED AND CELL-ASSOCIATED TISSUE FACTOR], James H. Morrissey et al., P. 247-261 | 1 - 13 |
| Y | THROMBOSIS RESEARCH, Vol. 50, No. 4, (1988), [RESOLUTION OF MONOMERIC AND HETERODIMERIC FORMS OF TISSUE FACTOR THE HIGH-AFFINITY CELLULAR RECEPTOR FOR FACTOR VII], J. T. Morrissey et al., P. 481-493 | 1 - 13 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| April 23, 1990 (23. 04. 90) | May 7, 1990 (07. 05. 90) |

| International Searching Authority | Signature of Authorized Officer |
|---|---|
| Japanese Patent Office | |

Form PCT ISA 210 (second sheet) (January 1985)

| | FURTHER INFORMATION CONTINUED FROM THE SECOND SHEET | |
|---|---|---|
| P | Biochemistry, Vol. 28, No. 20 (1989), [ Purification of Recombinant Human Tissue Factor ], Lisa R. Paborsky et al., P. 8072-8077 | 1 - 13 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers . , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers . , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

| | FURTHER INFORMATION CONTINUED FROM THE SECOND SHEET | |
|---|---|---|
| Y | THROMBOSIS RESEARCH, Vol. 55, No. 1, (1989), [ CORRELATION BETWEEN ANTIGENIC AND FUNCTIONAL EXPRESSION OF TISSUE FACTOR ON THE SURFACE OF CULTURED HUMAN EXDOTHELIAL CELLS FOLLOWING STIMULATION BY LIPOPOLY-SACCHARIDE ENDOTOXIN], Masashi Noguchi et al., P. 87-97 | 1 - 13 |
| Y | BLOOD, Vol. 72, No. 2, (1988), [ Monocyte-Associated Tissue Factor Is Suppressed by phorbol Myristate Acetate], John P. Brozna et al., p. 456-462 | 1 - 13 |

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers          , because they relate to subject matter not required to be searched by this Authority, namely:

2.☐ Claim numbers          , because they relate to parts of the international application that do not comply with the prescribed requirements to such an extent that no meaningful international search can be carried out, specifically:

3.☐ Claim numbers          , because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)